(19) Europäisches Patentamt
European Patent Office
Office européen des brevets

(11) Publication number: **0 409 296 A2**

# (12) EUROPEAN PATENT APPLICATION

(21) Application number: 90201585.8

(22) Date of filing: 19.06.90

(51) Int. Cl.[5]: C07J 71/00, A61K 31/58

(30) Priority: 20.07.89 GB 8916635

(43) Date of publication of application: 23.01.91 Bulletin 91/04

(84) Designated Contracting States: DE GB IT

(71) Applicant: FARMITALIA CARLO ERBA S.r.L.
Via Carlo Imbonati 24
I-20159 Milano(IT)

(72) Inventor: Bandiera, Tiziano
Corso Vittorio Emanuele 44
I-27025 Gambolo' (Pavia)(IT)
Inventor: Brambilla, Enzo
Via Togliatti 41b
I-22066 Mariano Comense (Como)(IT)
Inventor: Ferrari, Patrizia
Via Verdi 30
I-21100 Varese(IT)
Inventor: Gobbini, Mauro
Via Isonzo 14
I-21018 Sesto Calende (Varese)(IT)
Inventor: Mantegani, Sergio
Via Carlo Pisacane 57
I-20129 Milano(IT)
Inventor: Quadri, Maria Luisa
Via Carlo Porta 11
Cernusco sul Naviglio (Milano)(IT)
Inventor: Traquandi, Gabriella
Via Cilea 106
I-20151 Milano(IT)

(74) Representative: Ferrario, Vittorino
c/o Farmitalia Carlo Erba S.r.l. Via Carlo Imbonati 24
I-20159 Milano(IT)

## (54) Cardanolactam derivatives.

(57) Compounds of the formula I

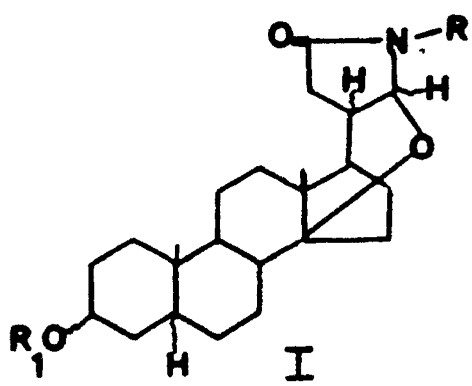

I

wherein R = H, optionally substituted $C_1$-$C_4$ alkyl, phenyl,benzyl, benzhydryl or trityl, $R_1$ = sugar residue, $(CHR_4)_nNR_2R_3$ n = 1,2,3; $R_2$ and $R_3$ = H, benzyl, $C_1$-$C_4$ alkyl or heterocycle ring when taken together with the nitrogen atom, and their pharmaceutically acceptable salt are useful as antihypertensive agents.

Their preparation and use as well as pharmaceutical composition containing them are also described.

EP 0 409 296 A2

## CARDANOLACTAM DERIVATIVES

The invention relates to cardanolactam derivatives, to a process for their preparation and to pharmaceutical compositions containing them.

The invention provides cardanolactam derivatives having the formula (I):

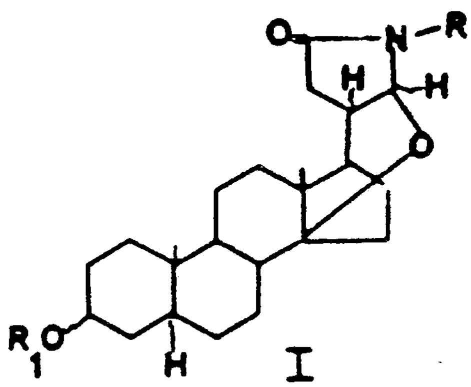

I

wherein R represents a hydrogen atom, $C_1$-$C_4$ alkyl,phenyl, benzyl, benzhydryl or trityl group unsubstituted or substituted by a $C_1$-$C_4$ alkoxy, carboxy, carbamoyl or di ($C_1$-$C_4$)alkylamino group; and $R_1$ represents a deoxy, dideoxy, aminodeoxy, aminodideoxy or aminotrideoxy sugar, or an aminoalkyl residue of the formula $-(CHR_4)_n-NR_2R_3$ wherein n represents 1, 2 or 3 and $R_2$ and $R_3$ independently represent a hydrogen atom, benzyl or $C_1$-$C_4$ alkyl group or, taken together with the nitrogen atom to which are linked, form a heterocycle residue which contains one or two heteroatoms selected from oxygen and nitrogen and which is optionally substituted by a $C_1$-$C_4$ alkyl group and $R_4$ represents hydrogen atom or taken together with $R_2$ or $R_3$ and the nitrogen atom to which they are linked forms a heterocycle residue as that above defined, optionally substituted by a $C_1$-$C_4$ alkyl group and the pharmaceutically acceptable salts thereof. The sugars which $R_1$ may represent include 6-deoxy-hexopyranosyl, 2,6-dideoxyhexopyranosyl, 2-amino or 2-alkylamino-2-deoxy-hexopyranosyl, 3-amino or 3-alkylamino 3-deoxy-hexopyranosyl, 3-amino or 3-alkylamino-3,6-dideoxyhexopyranosyl and 3-amino or 3-alkylamino-2,3,6-trideoxy-hexopyranosyl of the D or L series. and the pharmaceutically acceptable salts thereof.

A $C_1$-$C_4$ alkyl group includes methyl, ethyl, i-propyl and n-propyl groups. Preferably R is methyl. The heterocycle residue which $R_2$ and $R_3$ may represent with the nitrogen atom to which they are linked is preferably of the formula

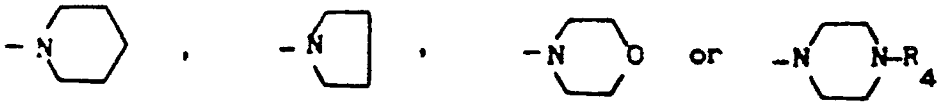

, , or

wherein $R_4$ represents a $C_1$-$C_4$ alkyl.

The wavy lines in the formula indicate that the substituents or the hydrogen atoms may be above or under the plane of the ring system providing optically active isomer forms having different absolute configurations. These optically active forms are encompassed by this invention.

Preferably it is possible to obtain optically active compounds of formula I by utilizing starting materials which are optically active.

"Pharmaceutically acceptable salts" refer to those salts which retain the biological effectiveness and properties of the free bases and which are not biologically or otherwise undesirable. Such salts are formed with inorganic acids such as hydrochloric, hydrobromic, sulfuric, nitric or phosphoric acid and organic acids such as acetic, propionic, glycolic, pyruvic, oxalic, malic, malonic, succinic, maleic, fumaric, tartaric, citric, benzoic, cinnamic, mandelic, methanesulfonic, ethanesulfonic, p-toluene sulfonic or salicyclic acid.

The invention further provides a process for the preparation of the cardanolactam derivatives of the formula (I) or a pharmaceutically acceptable salt thereof, which process comprises condensing a compound having the formula (II)

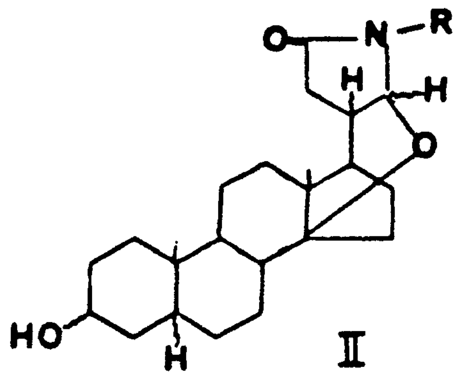

II

wherein R is as above defined, with an amino alkyl or sugar halide of the formula III : A - W, wherein W is a sugar residue as defined above or $-(CHR_4)_n-NR_2R_3$ III

wherein n,$R_2$,$R_3$and $R_4$ are as above defined, and A represents a halogen atom; and, if desired, converting the resulting cardanolactam derivative of formula (I) into a pharmaceutically acceptable salt thereof.

The condensation process is desirably carried out in a suitable organic solvent such as tetrahydrofuran, dioxane or dimethylformamide, in the presence of sodium hydride, at a temperature of from 70° to 130°C. Preferably, A represents a chlorine or bromine atom. After the usual work-up the ethers (I) are isolated as such or as an easily crystallizable salt, such as the oxalate.

The amino alkylhalides or sugar derivatives of the formula (III) used as starting materials are well known compounds. The cardanolactams of the formula (II) maybe prepared according to or in analogy to the procedure described by Deghenghi et al. (Can. J. Chem., 49 , 2676, 1971) or by N-alkylation of the unsubstituted cardanolactams (ibidem).

The cardanolactam derivatives according to the invention and their pharmaceutically acceptable salts are capable of inhibiting specific ouabain binding without inhibiting $Na^+$, $K^+$-ATPase activity and thus they may be useful agent for the treatment of hypertension.

The compounds of the invention can be administered in a variety of dosage forms, e.g. orally, in the form of tablets, capsules, sugar or film coated tablets, liquid solutions or suspensions; rectally, in the form of suppositories, parenterally, e.g. intramuscularly, or by intravenous injection or infusion; or topically. The dosage depends on the age, weight, conditions of the patient and administration route.

The invention includes pharmaceutical compositions comprising a compound of the invention in association with a pharmaceutically acceptable excipient (which can be a carrier or diluent). The pharmaceutical compositions containing the compounds of the invention are usually prepared following conventional methods and are administered in a pharmaceutically suitable form.

For example, the solid oral forms may contain, together with the active compound, diluents, e.g. lactose, dextrose, saccharose, cellulose, corn starch or potato starch; lubricants, e.g. silica, talc, stearic acid, magnesium or calcium stearate; and/or polyethylene glycols; binding agents e.g. starches, arabic gums, gelatin, methylcellulose, carboxymethylcellulose or polyvinyl pyrrolidone; disaggregating agents, e.g. a starch, alginic acid, alginates or sodium starch glycolate; effervescing mixtures; dyestuff; sweeteners; wetting agents, such as lecithin, polysorbates, laurysulphates; and, in general, non-toxic and pharmacologically inactive substances used in pharmaceutical formulations. These pharmaceutical preparations may be manufactured in known manner, for example, by means of mixing, granulating, tabletting, sugar-coating, or film-coating processes.

The liquid dispersions for oral administration may be e.g. syrups, emulsions and suspensions. The syrup may contain as carrier, for example, saccharose or saccharose with glycerine and/or manitol and/or sorbitol. The suspensions and the emulsions may contain as carrier, for example, a natural gum, agar, sodium alginate, pectin, methylcellulose, carboxymethylcellulose, or polyvinyl alcohol.

The suspensions or solutions for intramuscular injections may contain, together with the active compound, a pharmaceutically acceptable carrier, e.g. sterile water, olive oil, ethyloleate, glycols, e.g. propylene glycol, and, if desired, a suitable amount of lidocaine hydrochloride.

The solutions for intravenous injections or infusion may contain as carrier, for example, sterile water or, preferably, they may be in the form of sterile, aqueous, isotonic saline solutions.

The suppositories may contain together with the active compound a pharmaceutically acceptable carrier, e.g. cocoa-butter, a polyethylene glycol, a polyoxyethylene sorbitan fatty acid ester surfactant or lecithin. Compositions for topical application such as, creams, lotions or pastes, may be prepared by

admixing the active ingredient, with a conventional oleaginous or emulsifying excipient.

The following assays were carried out on the compounds of the invention.

"In vitro" assays to test the ability of cardanolactam derivatives of formula I to displace specific ouabain binding to the ($Na^+$-$K^+$)-ATPase receptors without inhibiting the ($Na^+$-$K^+$)-ATPase enzymatic activity.

Radiochemical assay:

A microsomial fraction enriched in ($Na^+$ -$K^+$)-ATPase was prepared from dog kidney outer medulla, according to Jorgensen (BBA 356: 36-52, 1974). The partially purified enzyme (0.5 ug of protein) was incubated in 3 mM $MgCl_2$, 3 mM EGTA, 80 mM Hepes buffer (pH 7.4) and 2 mM $y^2$ - $P^{32}$ -ATP, final volume 110 ul, at 37°C for 15 minutes with increasing concentrations of ouabain (as reference compound) or steroid ethers.

The reaction was stopped by the addition of 0.1 mM of cold perchloric acid (10% final concentration) and 0.5 ml of charcoal suspension (20% w/v). The suspension was centrifuged and the content of $^{32}P$ in the supernatant was measured by liquid scintillation counting (ref. Mall F. et al.; Biochem. Pharm. 33: No. 1, 47-53, 1984).

The effects of various concentrations of cardanolactams derivatives and ouabain were expressed as a percentage of inhibition of the total ($Na^+$-$K^+$)-ATPase activity and $IC_{50}$ values were calculated.

Displacement of ouabain ($H^3$) binding from human red blood cells

The procedure has been described elsewhere (Erdmann E. et al.; Arzneim. Forsh 34(II), no.10: 1314, 1984).

Washed erythrocytes (about 1-1.8 x $10^9$/ml) were incubated in 130 mM NaCl, 1 mM $MgCl_2$, 10 mM glucose, 10 mM sucrose, 10 mM Tris HCl buffer (pE 7.4) 2x$10^{-9}$M $^3$E ouabain and increasing concentration of the unlabelled cardanolactams derivatives at 3$^{-o}$C for 5 hours. Bound ouabain was quantitated by a rapid filtration technique (Whatman GF/C glass filter membranes) to separate free from membrane-bound ouabain. 'Whatman' is a Trade Mark. The radioactivity in the filters was determined by liquid scintillation counting. Non specific binding was defined as the binding in the presence of $10^{-3}$M unlabelled ouabain.

The dissociation constant ($E_D$ value) was calculated from the concentration of unlabelled cardanolactams derivatives which inhibit $^3$H-ouabain binding by 50% at equilibrium, by the method of Erdmann et al. (Schmiedeberg's Arch. Pharmacol. 283: 335, 1973).

Inhibition of $Na^+$efflux mediated by the ($Na^+$-$K^+$)-ATPase in human red blood cells

The procedure has been described elsewhere (Garay et al., Biochem. Pharmacol. 33:2013-2020, 1984). Washed red blood cells were suspended to a hematocrit of 20-25% in 74 mM $MgCl_2$, 2mM KCl, 84 mM sucrose, 10 mM MOPS/Tris buffer (pH 7.4 at 37°C) and 10 mM glucose.

Red cell suspensions were added in the cold to tubes containing $Mg^{++}$ sucrose-$K^+$ medium with increasing concentration of ouabain and fixed concentrations of cardanolactam derivatives The tubes were incubated at 37°C and aliquots of the suspensions were transferred to the cold and spun down at different times (0 - 10 - 20 - 30 minutes). External $Na^+$ concentrations were measured in the supernatants by atomic absorption. A kinetic analysis of the inhibition of ouabain sensitive $Na^+$ efflux as a function of different cardanolactam derivatives concentrations was done and the $IC_{50}$ for each compound was calculated.

'In vivo' assays to test the hypotensive activities of cardanolactam derivatives of formula I

Indirect measurements of systolic blood pressure was carried out in groups of 4 spontaneously hypertensive rats (SHR.Kyoto), 8 to 10 weeks of age, supplied by Charles Rives, Italy. The animals were maintained in an environment of 36°C for 10 to 15 minutes to allow pulse pressure to be recorded and then systolic blood pressure and heart rate were measured by the indirect tail cuff method using a W+W, BP recorder, model 8005. The compounds were given orally, suspended in 5% arabic gum, once a day for 4

consecutive days and measurements were carried out before beginning the treatment and 1 and 5 hours after dosing in both the first and fourth day of treatment. Control animals received the vehicle only (0.2 ml/100 g body weight). Drug induced changes in systolic blood pressure were calculated as differences from the pretreatment values.

The following Examples illustrate the invention.

## Example 1

(20S,21R)-3β-dimethylaminopropoxy-14,21-epoxy-5β,14β-N-methyl-20-cardanolactam (1).

To a 55-60% dispersion of sodium hydride in mineral oil (2.102 g - 0.048 mol) in 50 ml of dry THF was added 2 g (0.005 mol) of the (20S,21R)-3B-hydroxy-14,21-epoxy-5β,14β-N-methyl-20-cardanolactam in 50 ml of dry THF. It was stirred and refluxed under $N_2$ for 5 hours. 3-dimethylamino-1-propyl chloride (6.79 g - 0.056 mol) was added little by little to the reaction mixture. The system was stirred and refluxed for 20 hours. 100 ml of water was added little by little. The reaction mixture was concentrated in vacuo to remove THF. The mixture obtained was extracted with ethyl acetate. The organic phase was washed with water, dried over sodium sulfate, and evaporated in vacuo. The residue was stirred in hexane and filtered to give 1.79 g of the ether 1 as a white solid that was purified as acid oxalate, 1.8 g; m.p. 160-165° C (dec.). FD MS m/e: 472($M^+$). Rf ($CH_2Cl_2$/ /MeOH = 4/1) = 0.18. Detection with $Ce(SO_4)_2$ 2% in $H_2SO_4$ 1M.

## Example 2

(20R,21S)-3β-dimethylaminopropoxy-14,21-epoxy-5β,14β-N-methyl-20-cardanolactam.

This compound was prepared using the procedure of example 1, from 1 g (0.0026 mol) of (20R,21S)-3β -hydroxy-14,21-epoxy-5β,14β-N-methyl-20-cardanolactam, sodium hydride 55-60% (1.09 g - 0.025 mol) and 3.4 g (0.03 mol) of 3-dimethylamino-1-propylchloride in 80 ml of dry THF. There was obtained 0.9 g of (20R,21S)-3β-dimethylaminopropoxy-14β,21-epoxy-5β-N-methyl-20-cardanolactam, as foam.
MS m/e: 472 ($M^+$).Rf ($CH_2Cl_2$/MeOH = 4/1) =0.41. Detection with $Ce(SO_4)_2$ 2% in $H_2SO_4$ 1M.

## Example 3

(20S,21R)-3β-diethylaminoethoxy-14,21-epoxy-5β,14β-N-methyl-20-cardanolactam.

This compound was prepared using the procedure of example 1, from 0.464 g (0.0012 mol) of (20S,21R)-3β-hydroxy-14,21-epoxy-5β,14β-N-methyl-20-cardanolactam, sodium hydride 55-60% (1.152 g - 0.026 mol) and 4 g (0.03 mol) of 2-diethylamino-1-ethylchloride in 45 ml of dry THF. There was obtained 0.28 g of (20S,21R)-3β-diethylaminoethoxy-14,21-epoxy-5β,14β-N-methyl-20-cardanolactam, as foam.
MS m/e: 486 ($M^+$). Rf ($CH_2Cl_2$/MeOH = 9/1) = 0.11. Detection with $Ce(SO_4)_2$ 2% in $H_2SO_4$ 1M.

## Example 4

(20S,21R)-3β-dimethylaminoethoxy-14,21-epoxy-5β,14β-N-methyl-20-cardanolactam.

From 0.464 g (0.0012) of (20S,21R)-3β-hydroxy-14,21-epoxy-5β,14β-N-methyl-20-cardanolactam, 1.152 g (0.026 mol) of sodium hydride 55-60% and 3.2 g (0.03 mol) of 2-dimethylamino-1-ethylchloride in 45 ml of dry THF, as in the procedure of example 1, 0.2 g of (20S,21R)-3β-dimethylaminoethoxy-14,21-epoxy-5β,14β-N-methyl-20-cardanolactam was obtained, as foam.
MS m/e: 458 ($M^+$). Rf ($CH_2Cl_2$/MeOH = 9/1) = 0.06. Detection with $Ce(SO_4)_2$ 2% in $H_2SO_4$ 1M.

Example 5

(20S,21R)-3β-morpholinoethoxy-14,21-epoxy-5β,14β-N-methyl-20-cardanolactam.

This compound was prepared using the procedure of example 1, from 0.464 g (0.0012) of (20S,21R)-3β-hydroxy-14,21-epoxy-5β,14β-N-methyl-20-cardanolactam, 1.152 g (0.026 mol) of sodium hydride 55-60% and 4.5 g (0.03 mol) of 4-(2-chloroethyl)morpholine in 45 ml of dry THF. There was obtained 0.420 g (20S,21R)-3β-morpholinoethoxy-14,21-epoxy-5β,14β-N-methyl-20-cardano- lactam, as foam.
MS m/e: 500 ($M^+$). Rf ($CH_2Cl_2$/MeOH = 9/1) = 0.37. Detection with $Ce(SO_4)_2$ 2% in $H_2SO_4$ 1M.

Example 6

(20S,21R)-3β-(2-pyrrolidinoethoxy)-14,21-epoxy-5β,14β-N-methyl-20-cardanolactam.

From 0.464 g (0.0012 mol)of (20S,21R)-3β-hydroxy-14,21-epoxy-5β,14β-N-methyl-20-cardanolactam, 1.152 g (0.026 mol) of sodium hydride 55-60% and 4 g (0.03 mol) of 2-pyrrolidinoethylchloride in 45 ml of dry THF, as in the procedure of example 1, 0.42 g of (20S,21R)-3β-(2-pyrrolidinoethoxy)-14,21-epoxy-5β,14β-N-methyl-20-cardanolactam was obtained. m.p. 90-94° C.
MS m/e: 484 ($M^+$). Rf ($CHCl_3$/MeOH = 4/1) = 0.27. Detection with $Ce(SO_4)_2$ 2% in $H_2SO_4$ 1M.

Example 7

(20R,21S)-3β-(2-pyrrolidinoethoxy)-14,21-3-epoxy-5β,14β-N-methyl-20-cardanolactam.

From 0.464 g (0.0012 mol) of (20R,21S)-3β-hydroxy-14,21-epoxy-5β,14β-N-methyl-20-cardanolactanm, 1.152 g (0.026 mol) of sodium hydride 55-60% and 4 g (0.03 mol) of 2-pyrrolidinoethyl chloride in 45 ml of dry THF, as in the procedure of example 1, 0.39 g of (20R,21S)-3β-(2-pyrrolidinoethoxy)-14,21-epoxy-5β,14β-N-methyl-20-cardanolactam was obtained. m.p. 138-142° C. MS m/e: 484 ($M^+$). Rf ($CHCl_3$/ MeOH = 4/1) = 0.3. Detection with $Ce(SO_4)_2$ 2% in $H_2SO_4$ 1M.

Example 8

**(20S,21R)-3β-aminoethoxy-14,21-epoxy-5β,14α-N-methyl-20-cardanolactam**

Operating as in Example 1, but employing N-(2-bromoethyl)phthalimide, the title compound was obtained, after hydrazinolysis of the corresponding phtaliimido derivative, in 53% yield. m.p. 89°-91° C.

Example 9

**(20S,21R)-3β-(N-methyl-2(S)-pyrrolidyl)methyoxy-14,21-epoxy-5β,14α-N-methyl-20-cardanolactam**

Operating as in Example 2, but employing (N-methyl-2(S)-pyrrolidyl)chloromethyl hydrochloride, the title compound was obtained in 40% yield. m.p. 186°-188° C.

Example 10

**(20S,21R)-3α-dimethylaminopropoxy-14,21-epoxy-5β,14α-N-methyl-20-cardanolactam**

Operating as in Example 1, but employing (20S,21R)-3α-hydroxy-14,21-epoxy-5β,14α-N-methyl-20-cardanolactam, the title compound was obtained in 60% yield. m.p. 220°-224° C.

Example 11

**(20S,21R)-3β-aminopropoxy-14,21-epoxy-5β,14β-N-methyl-20-cardanolactam**

Operating as in Example 1, but using N-(3-bromopropyl)phthalimide, the title compound was obtained, after hydrazinolysis of the corresponding phthalimido derivative, in 35% yield. m.p. 157°-164° C.

Example 12

**(20S,21R)-3β-piperidinopropoxy-14,21-epoxy-5β,14β-N-methyl-20-cardanolactam**

Operating as in Example 1, but using N-(3-chloropropyl)piperidine, the title compound was obtained, 70% yield. m.p. 138°-141° C.

Example 13

**(20S,21R)3β-(N-methyl-2(S)-pyrrolidyl)methyloxy-14,21-epoxy-5β,14β-N-methyl-20-cardanolactam**

Operating as in Example 1, but employing (N-methyl-2(S)-pyrrolidyl)chloromethyl hydrochloride, the title compound was obtained in 60% yield. m.p. 165°-167° C.

Example 14

**(20S,21R)-3α-pyrrolidinoethoxy-14,21-epoxy-5β,14β-N-methyl-20-cardanolactam**

Operating as in Example 1, but employing (20S,21R)-3α-hydroxy-14,21-epoxy-5β,14β-N-methyl cardanolactam and N(2-chloroethyl)pyrrolidine, the title compound was obtained in 48% yield. m.p. 93°-95° C.

## Claims

1. A cardanolactam derivative having the formula (I):

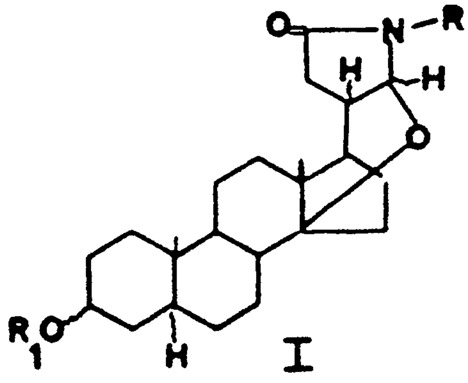

I

wherein R represents a hydrogen atom, $C_1$-$C_4$ alkylphenyl, benzyl, benzhydryl or trityl group unsubstituted or substituted by a $C_1$-$C_4$ alkoxy, carboxy, carbamoyl or di ($C_1$-$C_4$)alkylamino group; and $R_1$ represents a deoxy, dideoxy, aminodeoxy, aminodideoxy or aminotrideoxy sugar, or an aminoalkyl residue of the formula $-(CHR_4)_n-NR_2R_3$ wherein n represents 1, 2 or 3 and $R_2$ and $R_3$ independently represent a hydrogen atom, benzyl or $C_1$-$C_4$ alkyl group or, taken together with the nitrogen atom to which are linked, form a heterocycle residue which contains one or two heteroatoms selected from oxygen and nitrogen and which is optionally substituted by a $C_1$-$C_4$ alkyl group and $R_4$ represents hydrogen atom or taken together with $R_2$ or $R_3$ and the nitrogen atom to which they are linked forms a heterocycle residue as that above defined, optionally substituted by a $C_1$-$C_4$ alkyl group and the pharmaceutically acceptable salts thereof.

2. A compound according to claim 1, wherein R is methyl.

3. A compound according to claim 1 or 2, wherein $R_2$ and $R_3$, taken together with the nitrogen atom to which they are linked, form a heterocycle of the formula:

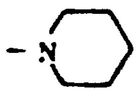 , 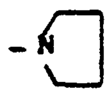 , 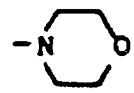 or 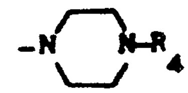

wherein $R_4$ represents a $C_1$-$C_4$ alkyl group.

4. A compound according to claim 1, which is selected from:
(20S,21R)-3β-dimethylaminopropoxy-14,21-epoxy-5β,14β-N-methyl-20-cardanolactam,
(20R,21S)-3β-dimethylaminopropoxy-14,21-epoxy-5β,14β-N-methyl-20-cardanolactam,
(20S,21R)-3β-diethylaminoethoxy-14,21-epoxy-5β,14β-N-methyl-20-cardanolactam,
(20S,21R)-3β-dimethylaminoethoxy-14,21-epoxy-5β,14β-N-methyl-20-cardanolactam,
(20S,21R)-3β-morpholinoethoxy-14,21-epoxy-5β,14β-N-methyl-20-cardanolactam,
(20S,21R)-3β-(2-pyrrolidinoethoxy)-14,21-epoxy-5β,14β-N-methyl-20-cardanolactam, and
(20R,21S)-3β-(2-pyrrolidinoethyoxy)-14,21-epoxy-5β,14β-N-methyl-20-cardanolactam.

5. A process for the preparation of a cardanolactam derivative of formula (I) as defined in claim 1 or a pharmaceutically acceptable salt thereof, which process comprises condensing a compound having the formula (II)

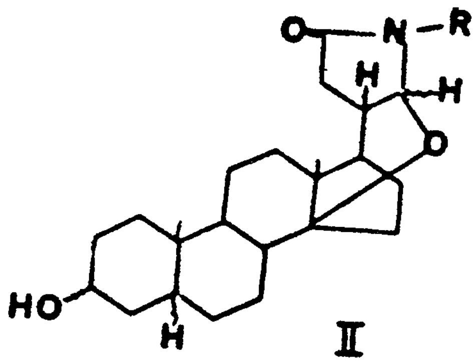

II

wherein R is as defined in claim 1, with an amino alkyl or sugar halide of the formula (III) A - W, wherein W is a sugar residue as defined in claim 1 or $-(CHR_4)_n-NR_2R_3$ (III), wherein n, $R_2$, $R_3$ and $R_4$ are as defined in claim 1 and A represents a halogen atom; and, if desired, converting the resulting cardanolactam derivative of formula (I) into a pharmaceutically acceptable salt thereof.

6. A process according to claim 5, wherein the condensation is carried out in an organic solvent, in the presence of sodium hydride, at a temperature of from 70° to 130°C.

7. A process according to claim 5 or 6, wherein A represents a chlorine or bromine atom.

8. A pharmaceutical composition comprising a pharmaceutically acceptable carrier of diluent and, as active ingredient, a cardanolactam derivative of formula (I) or pharmaceutically acceptable salt thereof as claimed in any one of claims 1 to 4 or which has been prepared by a process as claimed in any one of claims 5 to 7.

9. A cardanolactam derivative of formula (I) as defined in claim 1 or pharmaceutically acceptable salt thereof for use in a method of treatment of the human or animal body by therapy.

10. A compound according to claim 9, for use in the treatment of hypertension.